# EUROPEAN PATENT SPECIFICATION

(11) **EP 1 507 531 B1**
(45) Date of publication and mention of the grant of the patent: **24.01.2007**
(21) Application number: 04720451.6
(22) Date of filing: 12.03.2004
(51) Int. Cl.: A61K 31/4545, A61P 37/06

(54) **STABLE PHARMACEUTICAL COMPOSITIONS OF DESLORATADINE**
STABILE PHARMAZEUTISCHE ZUSAMMENSETZUNGEN MIT DESLORATADINE
COMPOSITIONS PHARMACEUTIQUES STABLES DE DESLORATADINE

(30) Priority: 12.03.2003 US 454299 P; 28.10.2003 US 515354 P; 03.11.2003 US 516904 P; 01.12.2003 US 526339 P
(43) Date of publication of application: 23.02.2005
(73) Proprietor: Teva Pharmaceutical Industries Ltd, 49131 Petah Tiqva (IL)
(72) Inventor: TOTH, Zoltan G., 4032 Debrecen (HU); GYOLLAI, Viktor, 4032 Debrecen (HU); KOVACSNE-MEZEI, Adrienne, 4032 Debrecen (HU); SZABO, Csaba, 4031 Debrecen (HU); ARONHIME, Judith, 76217 Rehovot (IL); SINGER, Claude, Kfar Saba 44358 (IL)
(74) Representative: Gallagher, Kirk James
(86) International application number: PCT/US2004/007723
(87) International publication number: WO 2004/080461

(56) References cited:
- EP-A- 0 270 818
- WO-A-95/10515
- WO-A-99/01450
- WO-A-03/086275
- WO-A-20/04012738
- US-A- 4 659 716
- US-B1- 6 506 767

## Description

### PRIORITY

This application claims the benefit of U.S. provisional application Serial No. 60/526,339, filed December 1, 2003, U.S. provisional application Serial No. 60/516,904, filed November 3, 2003, U.S. provisional application Serial No. 60/515,354, filed October 28, 2003, and U.S. provisional application Serial No. 60/454,299, filed March 12, 2003.

### FIELD OF THE INVENTION

The present invention relates to pharmaceutical compositions of desloratadine.

### BACKGROUND OF THE INVENTION

Desloratadine, known as 8-chloro-6,11-dihydro-11-(4-piperidylidene)-5H-benzo[5,6] cyclohepta[1,2-b]pyridine, has the following structure: 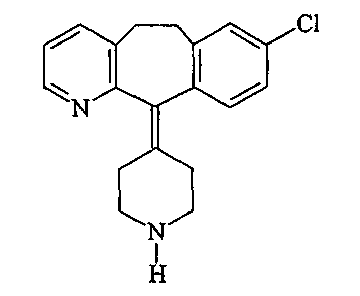 and is disclosed in U.S. Pat. No. 4,659,716. Desloratadine is currently marketed as Clarinex^{®} in the United States. Clarinex is prescribed as an antihistamine for prevention or treatment of allergenic reactions, which may result in symptoms such as sneezing, itchy eyes and hives. The '716 patent discloses methods for preparing and administering desloratadine and its pharmaceutically acceptable salts. *See also* U.S. Pat. No. 4,282,233, which discloses loratadine.

The present invention relates to the solid state physical properties of desloratadine. These properties can be influenced by controlling the conditions under which desloratadine is obtained in solid form. Solid state physical properties include, for example, the flowability of the milled solid. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Another important solid state property of a pharmaceutical compound is its rate of dissolution in aqueous fluid. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. The solid state form of a compound may also affect its behavior on compaction and its storage stability.

These practical physical characteristics are influenced by the conformation and orientation of molecules in the unit cell, which defines a particular polymorphic form of a substance. The polymorphic form may give rise to thermal behavior different from that of the amorphous material or another polymorphic form. Thermal behavior is measured in the laboratory by such techniques as capillary melting point, thermogravimetric analysis (TGA) and differential scanning calorimetry (DSC) and can be used to distinguish some polymorphic forms from others. A particular polymorphic form may also give rise to distinct spectroscopic properties that may be detectable by powder X-ray crystallography, solid state ¹³C NMR spectrometry and infrared spectrometry.

In Example V, the '716 patent prepares desloratadine in the solid state and discloses: "Extract the organic material with chloroform, wash with water and remove the solvent. Triturate the residue with hexane. Recrystallize from a large volume of hexane after charcoal decolorization to obtain the product, m.p. 151°-152°C."

In Example VI, B, desloratadine is also prepared in the solid state: "The material is extracted several times with chloroform, the chloroform extracts washed with water and concentrated to dryness, and the residue triturated with petroleum ether or hexane to yield 11.5 grams (93%) m.p. 149°-151°C. After recrystallization from hexane, the product melts at 150°-151°C." The starting material for Example VI, B, is an N-cyano compound prepared according to the disclosure in U.S. Pat. No. 3,326,924.

U.S. Pat. No. 6,506,767 discloses two polymorphic forms of desloratadine, labelled Forms I and II (*syn.* form 1 and form 2). The XRPD peaks and the FTIR spectrum for the forms are also disclosed in the `767 patent.

The '767 patent discloses: "Surprisingly we discovered that certain alcoholic solvents, e.g., hexanol and methanol produced 100% polymorph form 1, but others, e.g., 3-methyl-1-butanol and cyclohexanol produced significant amounts of form 2. Chlorinated solvents, e.g., dichloromethane produced form 1 substantially free of form 2 but the compounds were discolored. Ether solvents such as dioxane produced form 1 substantially free of form 2 but other alkane ethers" e.g., di-isopropyl ether produced form 1 with significant amounts of form 2 and di-n-butyl ether favored formation of form 2. Ketones such as methyl isobutyl ketone produced crystalline polymorph form 1 essentially free of form 2 but methyl butyl ketone produced a 8:1 ratio of form 1 to form 2. Use of methyl isubutyl ketone is preferred to produce crystalline polymorph form 1 essentially free of form 2. Only ethyl acetate and di-n-butyl ether were found to produce crystalline polymorph form 2 substantially free of form 1. Use of di-n-butyl ether is preferred for producing crystalline form 2 substantially free of fom 1." The '767 patent, in Examples 1-3, prepares Form I by crystallization out of methyl isobutyl ketone, while in examples 4 and 5, prepares Form II by crystallization out of ethyl acetate and di-n-butyl ether, respectively.

The '767 patent also carried out stability tests on Polymorph Form I. According to the '767 patent, Form I was "subjected to stability testing at various temperatures (25, 30 and 40°C) and relative humidities of 60%, 60% and 75%, respectively...No significant change (<1%) from initial sample % form 1 and related compounds was observed."

The `767 patent warns against using polymorphic mixtures of desloraratadine for formulation. According to the `767 patent, "such a mixture could lead to production of a [desloratadine] which would exist as a variable mixture of variable composition (i.e., variable percent amounts of polymorphs) having variable physical properties, a situation unacceptable in view of stringent GMP requirements."

There is a need in the art for additional pharmaceutical compositions of desloratadine.

### SUMMARY OF THE INVENTION

In one aspect, the present invention provides a pharmaceutical composition of desloratadine comprising of a mixture of crystalline form desloratadine I and II in a weight to weight ratio of 25% to 75% of either form to the other and a pharmaceutically acceptable excipient. The ratio can be 50%.

In another aspect, the present invention provides for a pharmaceutical composition of desloratadine comprising of crystalline form desloratadine I and II in a weight to weight ratio of 20% to 40% of Form II and a pharmaceutically acceptable excipient.

In another aspect, the present invention provides for a pharmaceutical composition of desloratadine prepared by a process comprising the steps of preparing a mixture of crystalline form desloratadine I and II in a weight to weight ratio of 20% to 40% Form II (Or up to 75% each) to Form I and combining the mixture with a pharmaceutically acceptable excipient to obtain a pharmaceutical composition.

In another aspect, the present invention provides for a stable mixture of crystalline form desloratadine I and II in a weight to weight ratio of 25% to 75% of either form to the other.

In another aspect, the present invention provides for a stable mixture of crystalline form desloratadine in a weight to weight ratio of from 20-40% Form II to 60-80% Form I.

In another aspect the present invention provides a stable mixture, and pharmaceutical compositions thereof, of crystalline form desloratadine I and II in a weight to weight ratio of 25% to 75% of either form, prepared by a process comprising:
a) combining desloratadine salt, toluene and a base to obtain a reaction mixture;
b) heating the mixture, whereby two phases are obtained;
c) separating the phases;
d) concentrating the separated organic phase;
e) dissolving the obtained concentrate in a toluene-2-propanol mixture containing less than 20% 2-propanol by volume;
f) cooling the solution to obtain a slurry;
g) combining the slurry with cold n-heptane; and
h) recovering mixture of desloratadine forms I and II.

### BRIEF DESCRIPTION OF THE FIGURE

Figure 1 is a stability study of a polymorphic mixture of desloratadine.

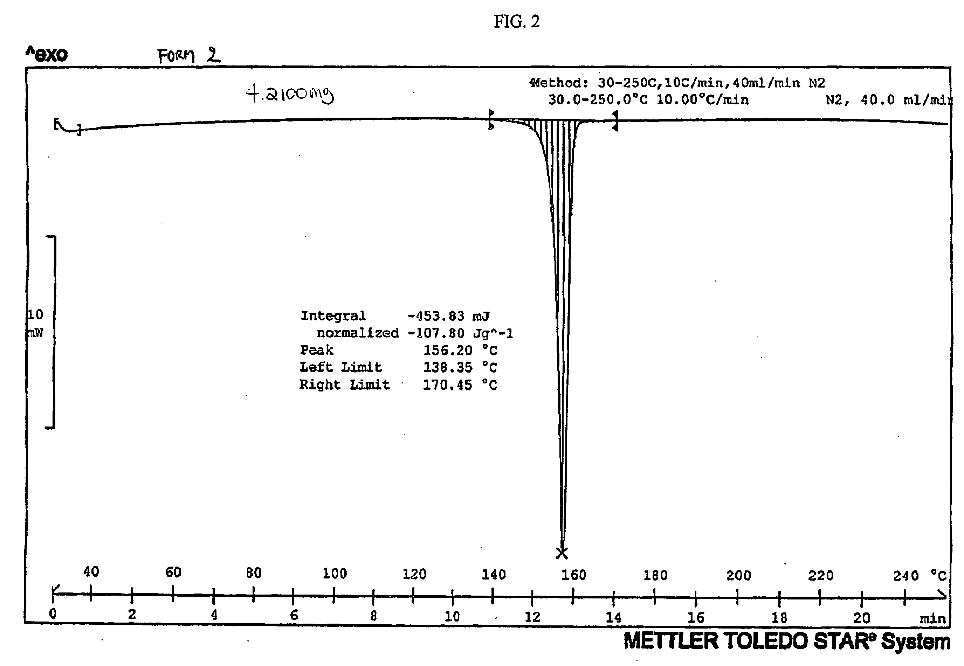

Figure 2 is a DSC thermogram of desloratadine Form II after grinding and sieving.

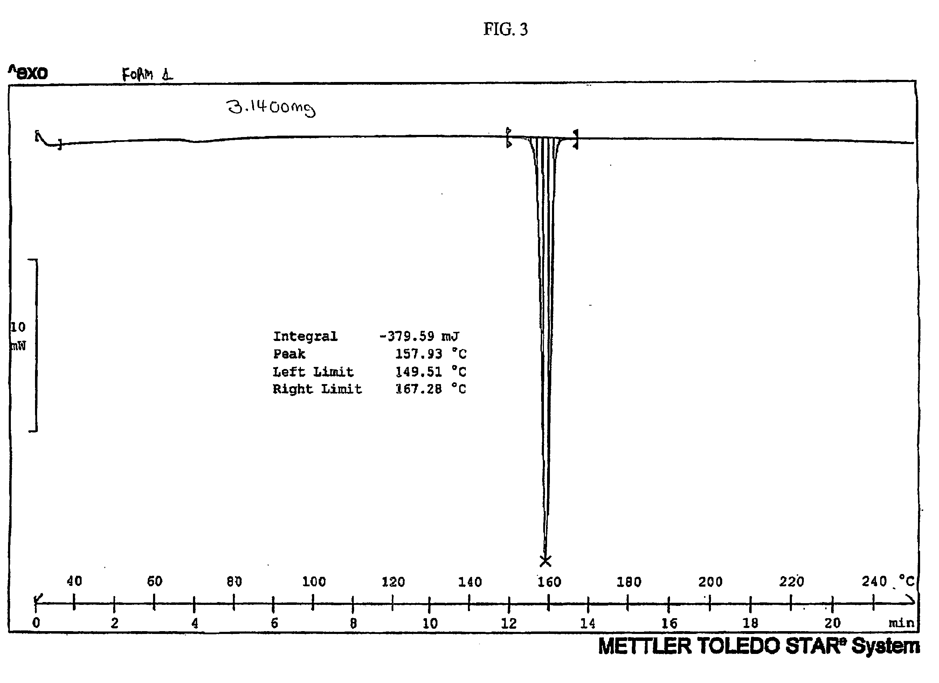

Figure 3 is a DSC thermogram of desloratadine Form I after grinding and sieving.

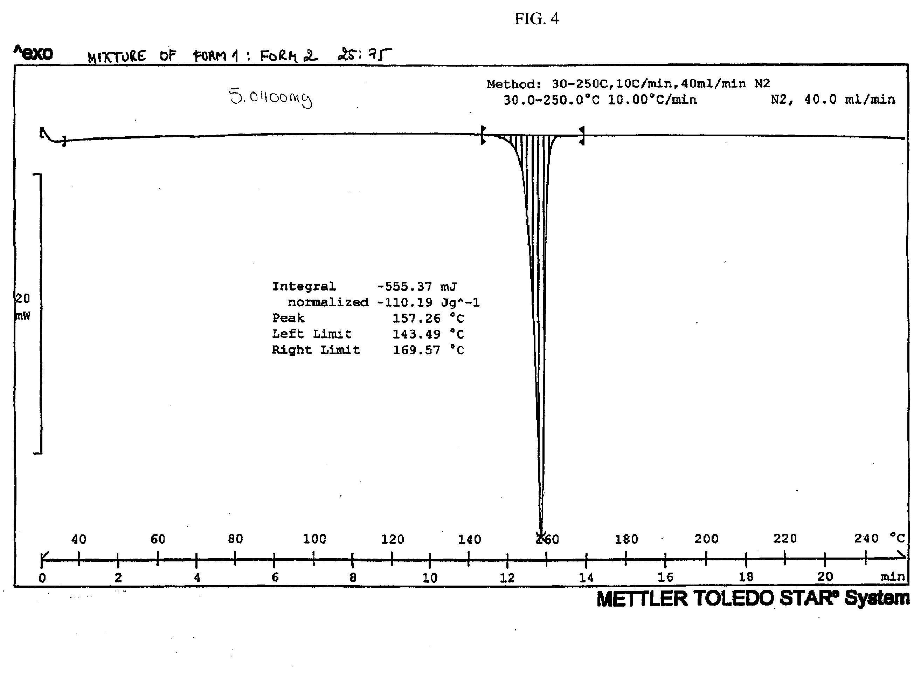

Figure 4 is a DSC thermogram of a 25:75 mixture of Form I and Form II by weight.

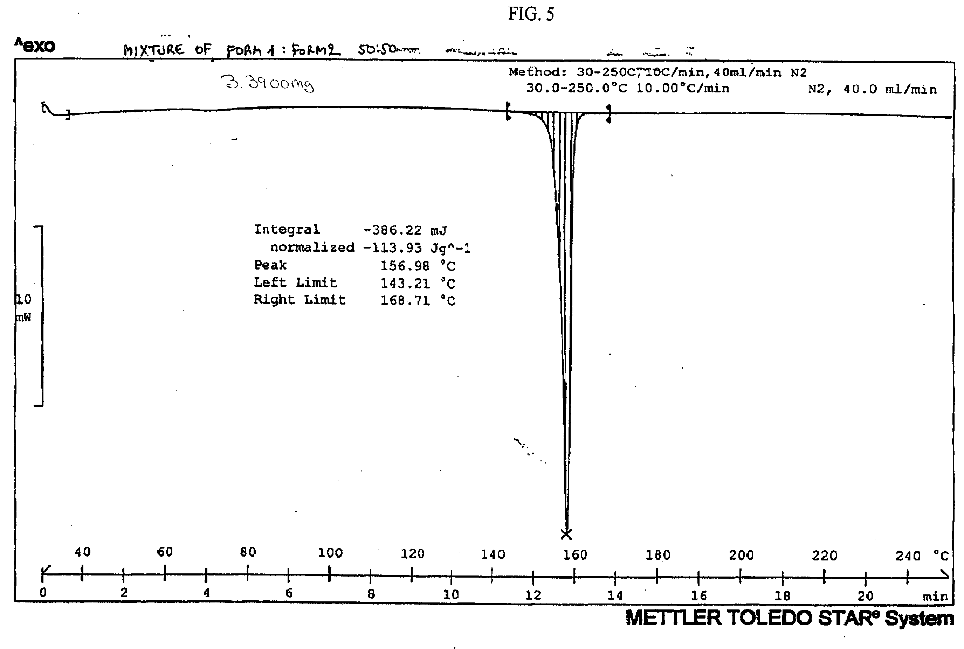

Figure 5 is a DSC thermogram of a 50:50 mixture of Form I and Form II by weight.

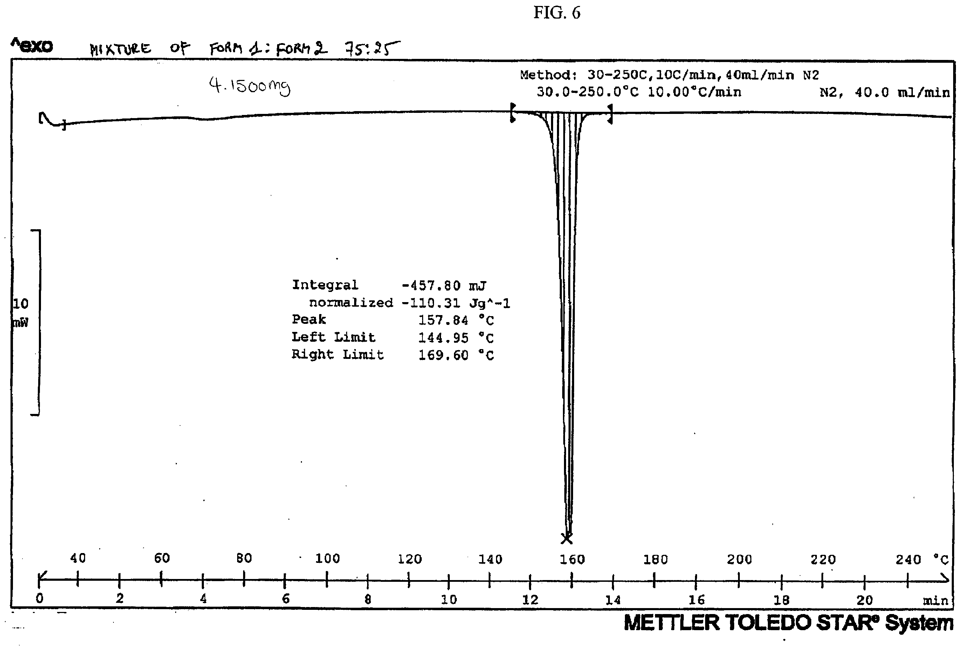

Figure 6 is a DSC thermogram of a 75:25 mixture of Form I and Form II by weight.

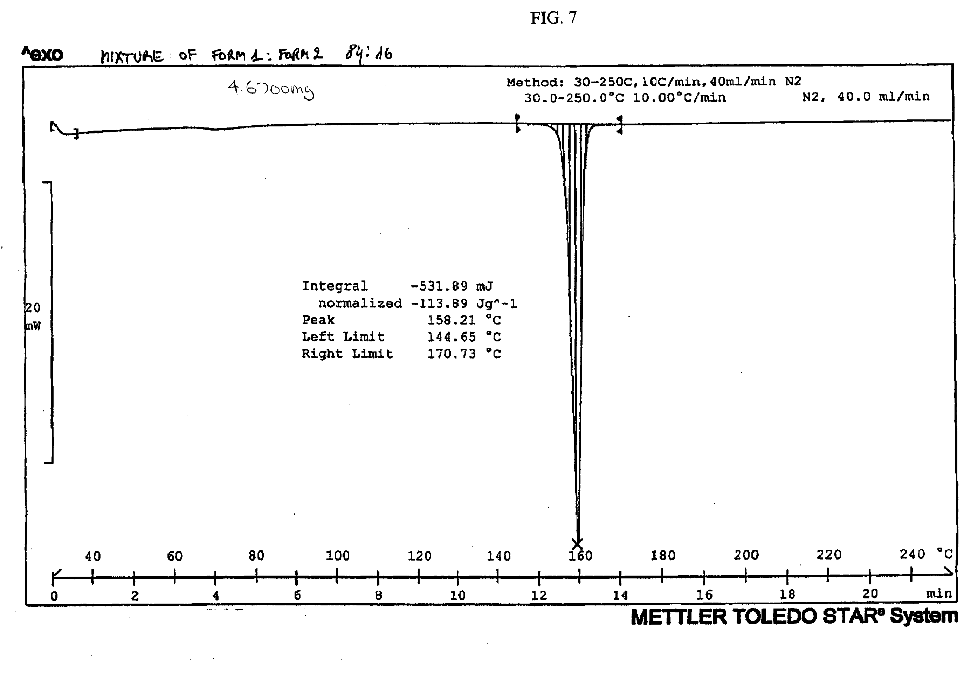

Figure 7 is a DSC thermogram of a 84:16 mixture of Form I and Form II by weight.

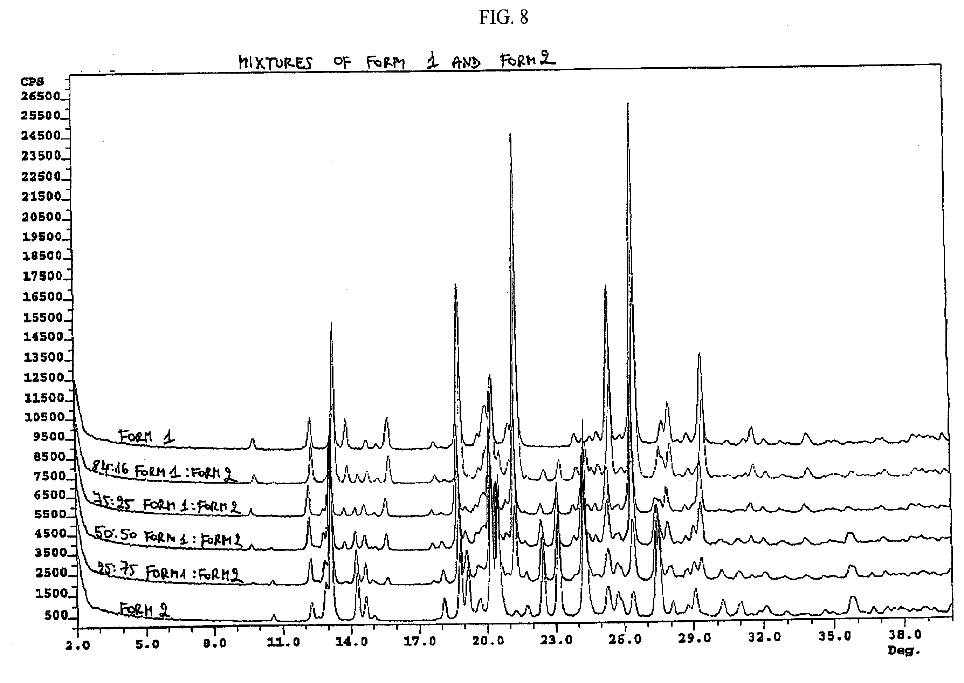

Figure 8 is a comparison of X-ray powder diffraction patterns of desloratadine Form I and Form II, and various mixtures thereof.

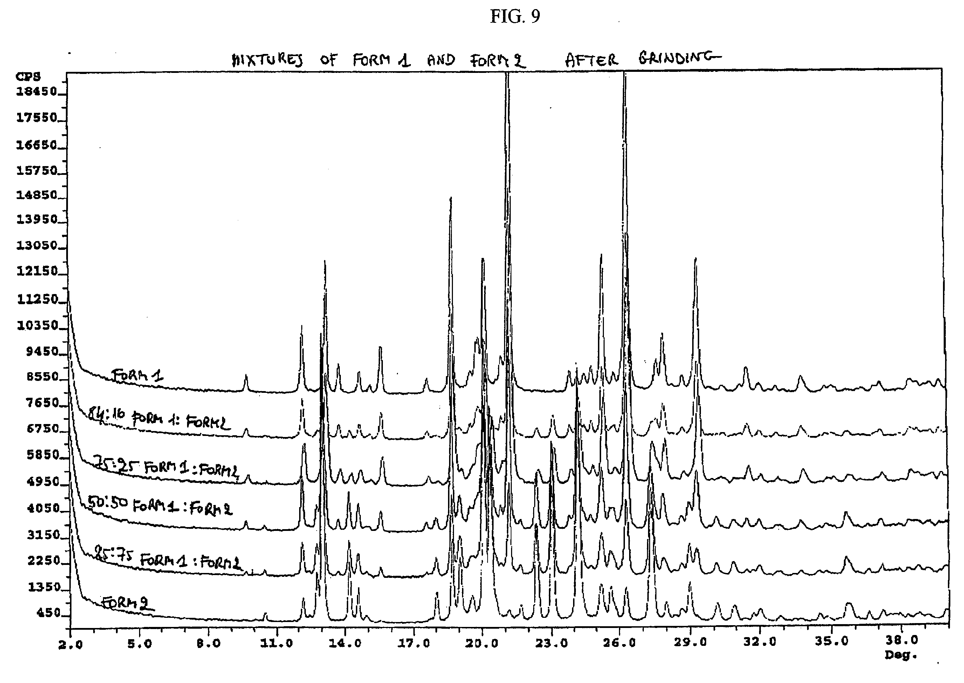

Figure 9 is similar to figure 8, but illustrates the X-ray diffraction patterns after grinding.

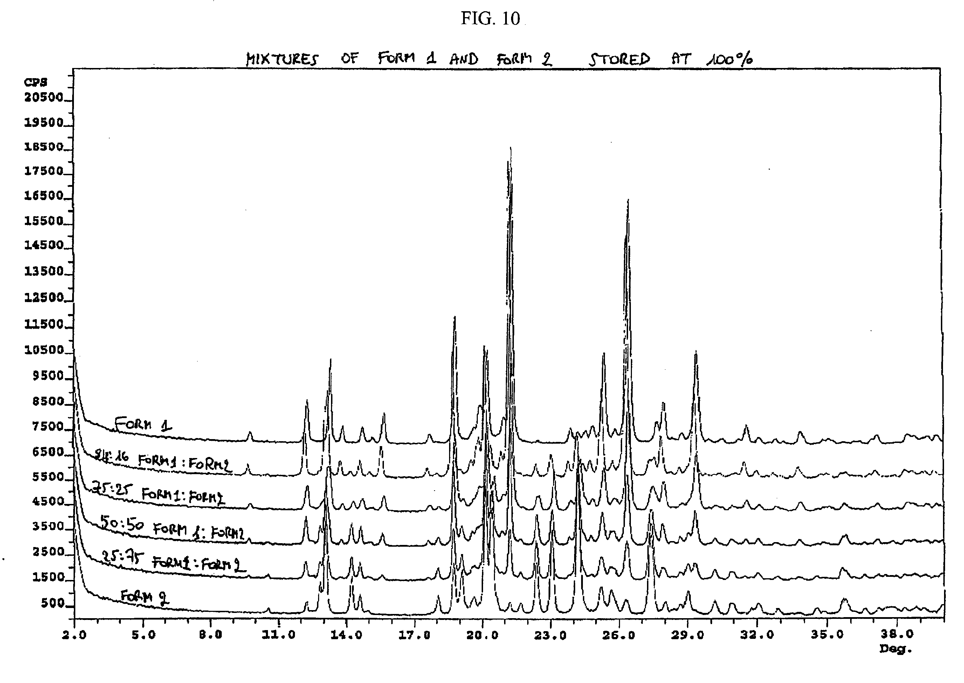

Figure 10 is similar to figure 8, but illustrates the X-ray diffraction patterns after storage at 100% relative humidity.

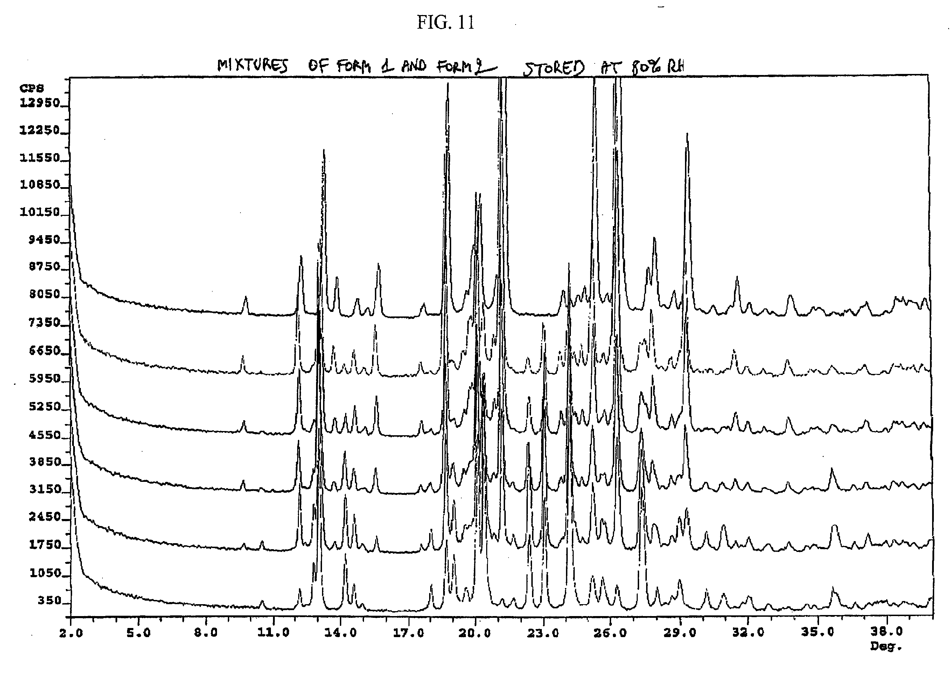

Figure 11 is similar to figure 8, but illustrates the X-ray diffraction patterns after storage at 80% relative humidity.

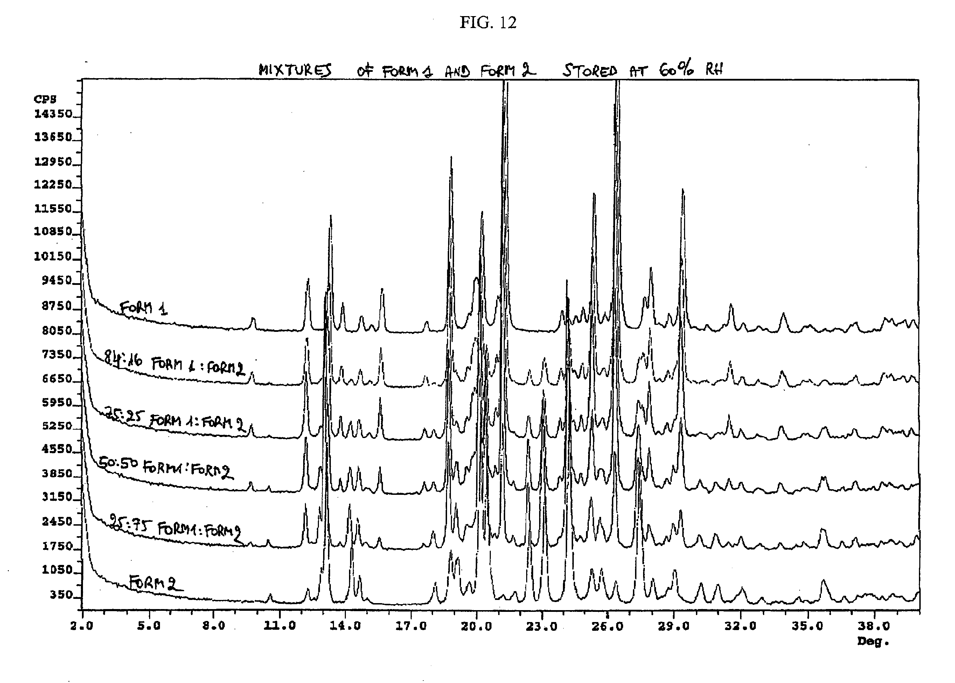

Figure 12 is similar to figure 8, but illustrates the X-ray diffraction patterns after storage at 60% relative humidity.

### DETAILED DESCRIPTION OF THE INVENTION

As used herein, the term drying refers to removal of solvent from a solid through application of heat.

The amount of Form I and Form II is expressed herein as a weight ratio relative to each other, *i.e.,* (Form I or II)/Form I plus Form II x 100%.

In the present invention provides a process suitable for industrial scale for preparation of formulations/compositions of desloratadine. Desloratadine may be crystallized as a mixture of polymorphs in such a way that the ratio between the polymorphs is consistent. As used herein, a "consistent ratio" (or consistent mixture) refers to a ratio of Form I compared to Form II (wt/wt) that is between a range of about ± 10% (wt/wt) between lots, as measured by XRPD or FTIR.

In one embodiment, the pharmaceutical composition is prepared by using a solution of desloratadine in toluene. The concentration of desloratadine is preferably at least 15% by weight. A salt of desloratadine may be used as starting material, particularly since salt formation may be used to purify the starting material. A suitable salt is the acetate salt.

When starting from a salt, depending on the solubility of the salt, the salt may be suspended in toluene as to form a slurry. A base is then added to the slurry to obtain the free acid, which is readily soluble in toluene, and moves into solution. Suitable bases include those of alkali metal and alkaline earth metals such as potassium, sodium and calcium oxide/hydroxide/carbonate, preferably sodium or potassium hydroxide.

The base is preferably added as an aqueous solution to the toluene, where two phases form. A 2% to 6% solution of sodium or potassium hydroxide, preferably about a 4% solution may be used. The slurry is preferably heated to increase the reaction rate, to for example a temperature of about 40 to about 70°C. The resulting two phase reaction system is preferably stirred at this temperature until complete dissolution.

The reaction results in neutralization of the salt, leading to solution of desloratadine free acid in toluene. After phase separation, such as by physical means with use of a separatory funnel, the toluene solution of desloratadine may be washed with distilled water at the same temperature to obtain more of the acid before discarding the aqueous phase.

In one embodiment, the resulting toluene solution is concentrated by vacuum or at atmospheric pressure (jacket: preferably about 55°C to about 130°C) to dryness, though it is theoretically possible to precipitate the acid by reducing the solubility of the solvent. The solid material is then dissolved in a mixture of toluene and 2-propanol, in the ratio of about 5:1 to about 15:1, more preferably about 9:1. The addition of relatively minor amounts of 2-propanol (anti-solvent) to toluene manipulates the ratio of Form I and II, and allows for a more facile crystallization. Without 2-propanol, substantially Form II is obtained rather than a mixture.

The mixture is preferably warmed to increase its solubility, such as to a temperature of about 50 to about 70°C, more preferably about 60°C. The warm solution is then preferably cooled to a temperature of about 10°C to about 30°C, more preferably to about 20°C. The cooling may be carried out slowly, during a span of few hours. Cooling in about 4 hours is optimal. After cooling, the resulting slurry is then preferably stirred for a few hours, more preferably of about 5 to about 8 hours.

This slurry is preferably warmed again, to about 45 to 55°C, and dropped into cold n-heptane, preferably at about -5 to about +5°C. The precipitated solid material is then recovered preferably by filtration, and may be dried. Drying may be carried out at ambient or reduced pressure. In one embodiment, drying is carried our in a vacuum oven at about 25-35°C overnight.

One skilled in the art may also appreciate that the present invention is not limited by the order of the additions in adding an anti-solvent. For example, a solution may be added to an anti-solvent or vice versa, though an embodiment may prefer one over the other. Crystallization of a compound is often better when a solution is added to the anti-solvent, but operationally it is often more convenient to add the anti-solvent to the solution. When adding an anti-solvent to a residue, the order of addition is of minimal relevance. The term combining encompasses both orders of addition.

The desloratadine used may be obtained from loratadine, by hydrolysis of the carbamate, preferably under basic conditions. Loratadine itself may be prepared from N-methyl desloratadine by removing N-methyl group of N-methyl desloratadine by formation of the carbamate through reaction with a haloformate. The haloformate used may be an alkyl or aryl formate, with optional halogen substituted at first and/or second position of the formate, *i.e.,* 2-chloroethyl-chloroformate. The carbamate may be prepared in an anhydrous C₅ to C₁₂ hydrocarbon, such as toluene. When N-methyl desloratadine is used as a stating material, loratadine may or may not be isolated in preparation of desloratadine.

The removal of the carbamate group of loratadine may be carried out with a base at elevated temperature. A preferred temperature is reflux temperature. A preferred base is an alkali metal or alkaline earth metal base such as potassium or sodium hydroxide. A preferred solvent is a C₁ to a C₄ alcohol such as 2-propanol.

The desloratadine from the reaction may then be recovered as a polymorphic form. In a preferred embodiment, the reaction mixture is distributed between an organic phase and water, resulting in desloratadine moving to the organic phase. The process described above with toluene may then be used, where a solution of desloratadine in toluene is prepared.

Pharmaceutical formulations of the present invention contain desloratadine Form I and/or Form II, optionally in mixture with other form(s) of desloratadine. The desloratadine prepared by the processes of the present invention are ideal for pharmaceutical composition. In addition to the active ingredient(s), the pharmaceutical compositions of the present invention may contain one or more excipients. Excipients are added to the composition for a variety of purposes.

Diluents increase the bulk of a solid pharmaceutical composition, and may make a pharmaceutical dosage form containing the composition easier for the patient and care giver to handle. Diluents for solid compositions include, for example, microcrystalline cellulose (e.g. Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

Glidants can be added to improve the flowability of a non-compacted solid composition and to improve the accuracy of dosing. Excipients that may function as glidants include colloidal silicon dioxide, magnesium trisilicate, powdered cellulose, starch, talc and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol and tartaric acid.

Solid and liquid compositions may also be dyed using any pharmaceutically acceptable colorant to improve their appearance and/or facilitate patient identification of the product and unit dosage level.

In liquid pharmaceutical compositions of the present invention, desloratadine and any other solid excipients are dissolved or suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

Sweetening agents such as sorbitol, saccharin, sodium saccharin, sucrose, aspartame, fructose, mannitol and invert sugar may be added to improve the taste.

Preservatives and chelating agents such as alcohol, sodium benzoate, butylated hydroxy toluene, butylated hydroxyanisole and ethylenediamine tetraacetic acid may be added at levels safe for ingestion to improve storage stability.

According to the present invention, a liquid composition may also contain a buffer such as guconic acid, lactic acid, citric acid or acetic acid, sodium guconate, sodium lactate, sodium citrate or sodium acetate. Selection of excipients and the amounts used may be readily determined by the formulation scientist based upon experience and consideration of standard procedures and reference works in the field.

The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable administration in any given case will depend on the nature and severity of the condition being treated, the most preferred route of the present invention is oral. The dosages may be conveniently presented in unit dosage form and prepared by any of the methods well-known in the pharmaceutical arts.

Dosage forms include solid dosage forms like tablets, powders, capsules, suppositories, sachets, troches and losenges, as well as liquid syrups, suspensions and elixirs.

The dosage form of the present invention may be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

The active ingredient and excipients may be formulated into compositions and dosage forms according to methods known in the art.

A composition for tableting or capsule filling may be prepared by wet granulation. In wet granulation, some or all of the active ingredients and excipients in powder form are blended and then further mixed in the presence of a liquid, typically water, that causes the powders to clump into granules. The granulate is screened and/or milled, dried and then screened and/or milled to the desired particle size. The granulate may then be tableted, or other excipients may be added prior to tableting, such as a glidant and/or a lubricant.

A tableting composition may be prepared conventionally by dry blending. For example, the blended composition of the actives and excipients may be compacted into a slug or a sheet and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting, however, they are not subjected to a final tableting step.

Capsules, tablets and lozenges, and other unit dosage forms preferably contain from about 2 to about 20 mg of desloratadine, more preferably about 2 mg to about 10 mg of desloratadine, and most preferably about 5mg.

The compositions of the present invention are substantially physically stable, *i.e.,* substantially stable against polymorphic transformations. The stable compositions may be manufactured in accordance with the acceptable GMP requirements. The stability tests below show the relative stability of the two forms for at least about two months under accelerated conditions. The mixtures are physically stable and undergo less than 10%, more preferably less than 5% and most preferably less than 3% polymorphic change per each polymorph after storage under accelerated ageing conditions (40°C and 75% RH) for at least 2 months. The mixtures also undergo less than 10%, more preferably less than 5% and most preferably less than 1% polymorphic change in each polymorph when stored for at least 2 months at room temperature and 60% RH. Additional stability may be imparted by formulating the desloratadine. The pharmaceutical formulation as a mixture may include stable mixtures of 25% to 75% weight/weight of one form compared to the other, such as 25% of Form I, 50% of Form I and 75% of Form I, with the rest Form II. In one embodiment, a 55-65% of Form I and 35-45 % of Form II mixture is used. In another embodiment about 20 to 40% of Form II is used, more preferably 24% to about 38%.

The term GMP requirement refers to consistency among batches. The physical properties concerning GMP requirements are stability and solubility. The dissolution rate was not tested due to very low solubility of desloratadine. Nevertheless, the dissolution rate of the stable mixture, when measured by the USP Paddle Method at 50-90 RPM in 900mL water is preferably not less than 80% by weight of the mixture released after 30 minutes. Preferably, the solubility of the bactches is within about a ±10%, more preferably within about ±5%, and most preferably within about ±1-3% compared to each other.

Stability of desloratadine at relative humidities of 60% 80% and 100% RH, stability under grinding, thermal stability/melting point in the DSC was monitored. The stable mixtures of 25:75, 50:50, 75:25 (falling under the present invention), 84:16 (reference mixture) (Form 1:Form 2) do not show any substantial change (Chemical: by degradation; Physical: by transformation to another polymorphic form) in the XRD pattern after exposure at 60%, 80%, 100% RH for one week. Also those stable mixtures do not show any substantial change in the XRD pattern after grinding for one minute; The sample is ground by hand in a mortar and pestle for about 1 minute. The separate polymorphs (Form I and Form II) were also monitored as a reference, and shown to be stable as well. The mixtures also show a substantial lack in chemical decomposition after storage at 100% humidity for one week and after grinding for 1 minute. This lack of decomposition is preferably undetectable by XRD and NMT 3%, more preferably NMT 2%, and most preferably NMT 3% by weight.

The physical properties of the two separate polymorphs (Form I and Form II) were compared to the physical properties of some mixtures (25:75, 50:50, 75:25 (falling under the present invention), 84:16 (reference mixture) Form I:Form II). It was discovered that polymorphic mixtures with different polymorphic compositions have practically invariable physical properties as compared to the separate polymorphs (Form I and Form II). Hence, even if there is polymorphic transformation, the thermal characteristics of the polymorphic mixture may remain substantially the same, which is ideal for formulation.

The thermal stability of the mixtures of polymorphs is comparable to that of the separate polymorphs. The melting temperatures of the stable mixtures, as determined in the DSC, is in the range of 157-158°C, while the separate polymorphs give in the DSC melting temperatures of 156°C and 158°C for Form II and Form I respectively. The similarity in melting points of the separate polymorphs and the stable mixtures indicates that the physical properties are not altered significantly. The DSC curves of the separate polymorphs also do not show any exotherm of decomposition at the temperature above the melting temperature, and also the stable mixtures do not show any event of decomposition above the melting temperature. This lack of decomposition indicates that the mixtures like the separate polymorphs are substantially thermally stable.

A particularly preferred range for the mixture of the present invention is 20% to 40% Form II compared to Form I.

The stable mixtures may be analysed by (FTIR) or X-Ray powder diffraction. Both techniques can be used to monitor polymorphic changes. X-Ray is reported in the literature for its capability to detect generally around 5% polymorphic impurities, but in many cases also to about 1% by weight. With FTIR however, the level of detection is not as accurate.

### Instrumentation

X-Ray powder diffraction data were obtained using by method known in the art using a SCINTAG powder X-Ray diffractometer model X'TRA equipped with a solid state detector. Copper radiation of 1.5418 Å was used. A round aluminum sample holder with round zero background quartz plate, with cavity of 25(diameter)*0.5(dept) mm.

DSC analysis was done using a Mettler 821 Star^{e}. The weight of the samples was about 5 mg; the samples were scanned at a rate of 10°C/min from 30°C to 250°C. The oven was constantly purged with nitrogen gas at a flow rate of 40 ml/min. Standard 40 µl aluminum crucibles covered by lids with 3 holes were used.

IR analysis was done using a Perkin Elmer SPECTRUM ONE FT-IR spectrometer in DRIFTt mode. The samples in the 4000-400 cm⁻¹ interval were scanned 64 times with 4.0 cm⁻¹ resolution

In the following examples, the vacuum oven used had a pressure of approximately 30 mm Hg and the refrigerator had a temperature of about 5 °C.

### EXAMPLE 1

### Preparation of desloratadine mixture Form I and Form II

A slurry of desloratadine acetate (20 grams) in toluene (100 ml) and 3.8% KOH solution (95,6 ml) was heated and stirred in the glass reactor at 60 °C until complete dissolution. After phase separation, toluene solution of desloratadine was washed with distilled water (60 ml) at 60°C. The resulting toluene solution was concentrated by vacuum (jacket: 60°C) to dryness. The solid material was dissolved in toluene-2-propanol 9:1 (74 ml) at 60°C. The warm solution was cooled to 20°C for 4 hours and stirred at this temperature for 8 hours. This slurry was warmed again to 45°C. In another glass reactor n-heptane (100 ml) was cooled to 0°C. The warm slurry of desloratadine in toluene was dropped into cold n-heptane (temperature of slurry was between 0-12°C), and it was stirred at 0°C for 1 hours. The resulting crystalline product was filtered and dried in a vacuum oven at room temperature. The X-Ray Powder Diffraction showed that the sample had crystallized in as a mixture of polymorphic Form I and Form II (ratio 76 - 24). (13.2 g, 79%, HPLC purity: 99.9 %).

### EXAMPLE 2

### Preparation of desloratadine mixture Form I and Form II

Desloratadine was prepared from desloratadine acetate according to the example 1. The X-Ray Powder Diffraction showed that the sample had crystallized in as a mixture of polymorphic Form I and Form II (10.8 g, 65%, HPLC purity: 99.8 %). Mixture of Form I and Form II was in the ratio of 42 to 38.

### EXAMPLE 3

### Preparation of desloratadine mixture Form I and Form II

A slurry of desloratadine acetate (20 grams) in toluene (100 ml) and 3.8% KOH solution (75 ml) was heated and stirred in the glass reactor at 60 °C until complete dissolution. After phase separation, toluene solution of desloratadine was washed with distilled water (60 ml) at 60°C. The resulting toluene solution was concentrated by vacuum (jacket: 60°C) to dryness. The solid material was dissolved in toluene-2-propanol 9:1 (50 ml) at 60°C. The warm solution was cooled to 20°C for 4 hours and stirred at this temperature for 10 hours. This slurry was warmed again to 50°C. The resulting toluene slurry was concentrated by vacuum (jacket: 50°C) to half of volume. In another glass reactor n-heptane (100 ml) was cooled to 0°C. The warm slurry of desloratadine in toluene was dropped into cold n-heptane (temperature of slurry was between 0-12°C), and it was stirred at 0°C for 1 hours. The resulting crystalline product was filtered and was dried in a vacuum oven at room temperature. The X-Ray Powder Diffraction showed that the sample had crystallized in as a mixture of polymorphic Form I and Form II (64 to 36%) (10.6 g, 63%) HPLC purity: 99.7 %.

### Study of Stability of Desloratadine Polymorphs

The desired mixture of polymorphic forms (Form 1 and Form 2) of desloratadine was prepared by different methods in order to investigate a stability of polymorphic ratio of desloratadine. Stability of ratio of the mixture of desloratadine polymorphic forms at two different conditions:
1.) 25°C, 60 % RH
2.) 40°C, 75 % RH

In all cases samples were packed in polyethylene and store above conditions.

### Example 1 of stability tests:

Recrystallization of desloratadine (3 g) from dimethyl carbonate-diethyl carbonate (1:1) (35 ml) at 110°C obtained a mixture. The X-ray Powder Diffraction measurement showed that the ratio of the two polymorphic forms was 57:43 (Form 1/Form 2).

| | | **25°C 60% RH** | | **40°C 75% RH** | |
|---|---|---|---|---|---|
| Time | Time (weeks ) | Form 1(%) | Form 2 (%) | Form 1 (%) | Form 2 (%) |
| 0 week | 0 | 57 | 43 | 57 | 43 |
| 1 week | 1 | 59 | 41 | 46 | 54 |
| 2 weeks | 2 | 53 | 47 | 43 | 57 |
| 1 month | 4 | 55 | 45 | 50 | 50 |
| 2 months | 8 | 63 | 37 | 39 | 61 |
| 3 months | 12 | 52 | 48 | | |

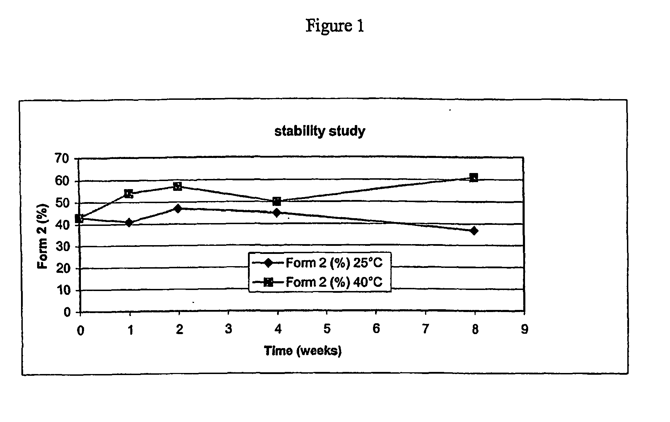

Stability study is disclosed in Figure 1.

### Example 2 of Stability Tests:

Recrystallization of desloratadine (3 g) from iso-propanol-n-heptane (1:1) (11 ml) at 85 °C obtained a mixture. The X-ray Powder Diffraction measurement showed that the ratio of the two polymorphic forms was 62:38 (Form 1/Form 2).

| | | **25 °C, 60% RH** | | **40 °C, 75% RH** | |
|---|---|---|---|---|---|
| Time | Time (weeks) | Form 1 (%) | Form 2 (%) | Form 1 (%) | Form 2 (%) |
| 0 week | 0 | 62 | 38 | 62 | 38 |
| 1 week | 1 | 65 | 35 | 63 | 37 |
| 2 weeks | 2 | 61 | 39 | 64 | 36 |
| 1 month | 4 | 66 | 34 | 65 | 35 |
| 2 months | 8 | 67 | 33 | 65 | 35 |
| 3 months | 12 | 66 | 34 | | |

### Example 3 of Stability Tests:

Recrystallization of desloratadine (10 g) from iso-propanol (25 ml) at 80 °C. The cooling term was regulated. The temperature was cooled to 0°C in 30 min, which generated a mixture. The X-ray Powder Diffraction measurement showed that the ratio of the two polymorphic forms was 74:26 (Form 1/Form 2).

| | | **25 °C, 60% RH** | | **40 °C, 75% RH** | |
|---|---|---|---|---|---|
| Time | Time (weeks) | Form 1 (%) | Form 2 (%) | Form 1 (%) | Form 2 (%) |
| 0 week | 0 | 74 | 26 | 74 | 26 |
| 1 week | 1 | 79 | 21 | 77 | 23 |
| 2 weeks | 2 | 74 | 26 | 81 | 19 |
| 1 month | 4 | 79 | 21 | 78 | 22 |
| 2 months | 8 | 75 | 25 | 78 | 22 |
| 3 months | 12 | 74 | 26 | 75 | 25 |
| 6 months | 24 | 77 | 23 | 75 | 25 |

### Example 4 of Stability Tests:

Recrystallization of desloratadine (10 g) from iso-propanol (25 ml) at 80 °C. The solution was seeded with Form II. The cooling term was regulated. The temperature was allowed to 0 °C in 30 minutes. The X-ray Powder Diffraction measurement showed that the ratio of the two polymorphic forms was 76:24 (Form 1/Form 2).

| | | 25 °C, 60% RH | | 40 °C, 75% RH | |
|---|---|---|---|---|---|
| Time | Time (weeks) | Form 1 (%) | Form 2 (%) | Form 1 (%) | Form 2 (%) |
| 0 week | 0 | 76 | 24 | 76 | 24 |
| 1 week | 1 | 87 | 13 | 76 | 24 |
| 2 weeks | 2 | 80 | 20 | 82 | 18 |
| 1 month | 4 | 82 | 18 | 88 | 12 |
| 2 months | 8 | 86 | 14 | 85 | 15 |
| 3 months | 12 | 81 | 19 | 82 | 18 |
| 6 months | 24 | 84 | 16 | 82 | 18 |

The Examples are set forth to aid in understanding the invention. The examples do not include detailed descriptions of conventional methods. Such methods are well known to those of ordinary skill in the art and are described in numerous publications. Polymorphism in Pharmaceutical Solids, Drugs and the Pharmaceutical Sciences, Volume 95 may be used as a guidance.

## Claims

1. A pharmaceutical composition of desloratadine comprising a mixture of crystalline form desloratadine I and II in a weight to weight ratio of 25% to 75% of either form to the other and a pharmaceutically acceptable excipient.

2. The pharmaceutical composition of claim 1, wherein the ratio is 50%.

3. The pharmaceutical composition of claim 1, wherein the ratio is 55 to 65% Form I to 35 to 45% of Form II.

4. A pharmaceutical composition of desloratadine comprising crystalline form desloratadine I and II in a weight to weight ratio of 20% to 40% of Form II and 60 to 80% Form I, and a pharmaceutically acceptable excipient.

5. The pharmaceutical composition of claim 4, wherein the Form II content of the mixture is 24% to 38%.

6. The pharmaceutical composition of any preceding claim, wherein the mixture used for the composition has a melting temperature of 157°C to 158°C as measured by DSC.

7. The pharmaceutical composition of any preceding claim, wherein the mixture used for the composition undergoes less than 1% by weight polymorphic change and chemical degradation after grinding for one minute.

8. The pharmaceutical composition of any preceding claim, wherein the mixture used for the composition undergoes less than 1% by weight chemical decomposition after storage at 100% relative humidity for one week.

9. The pharmaceutical composition of any preceding claim, wherein the mixture used for the composition undergoes less than 10% polymorphic change for each polymorph after storage for 2 months at 40°C at 75% RH.

10. The pharmaceutical composition of claim 9, wherein the mixture used for the composition undergoes less than 5% polymorphic change for each polymorph after storage for 2 months at 40°C at 75% RH.

11. The pharmaceutical composition of claim 10, wherein the mixture used for the composition undergoes less than 3% polymorphic change for each polymorph after storage for 2 months at 40°C at 75% RH.

12. The pharmaceutical composition of any preceding claim, wherein the mixture used for the composition undergoes less than 10% polymorphic change for each polymorph after storage for 2 months at room temperature at 60% RH.

13. The pharmaceutical composition of claim 12, wherein the mixture used for the composition undergoes less than 5% polymorphic change for each polymorph after storage for 2 months at room temperature at 60% RH.

14. The pharmaceutical composition of claim 13, wherein the mixture used for the composition undergoes less than 1% polymorphic change for each polymorph after storage for 2 months at room temperature at 60% RH.

15. Use of the pharmaceutical composition of any preceding claim in the preparation of a medicament for preventing or treating allergenic reactions in a mammal.

16. A process for preparing a pharmaceutical composition of desloratadine, said process comprising the steps of:
a) preparing a mixture of crystalline form desloratadine I and II in a weight to weight ratio of 20% to 40% Form II to Form I; and
b) combining the mixture with a pharmaceutically acceptable excipient to obtain a pharmaceutical composition.

17. A stable mixture of crystalline form desloratadine I and II in a weight to weight ratio of 25% to 75% of either form to the other.

18. A stable mixture of crystalline form desloratadine in a weight to weight ratio of from 20-40% Form II to 60-80% Form I.

19. The stable mixture of claim 18, wherein the weight to weight ratio is from 24-38% Form II to 62-76% Form I.

20. The stable mixture of any of claims 17 to 19 wherein the melting point is in the range of 157°C to 158°C.

21. The stable mixture of any one of claims 17 to 20, wherein the mixture is stable in that it undergoes less than 10% polymorphic change for each polymorph after storage for 2 months at 40°C at 75% RH.

22. The stable mixture of claim 21, wherein the mixture undergoes less than 5% polymorphic change for each polymorph after storage for 2 months at 40°C at 75% RH.

23. The stable mixture of claim 22, wherein the mixture undergoes less than 3% polymorphic change for each polymorph after storage for 2 months at 40°C at 75% RH.

24. The stable mixture of any of claims 17 to 23, wherein the mixture undergoes less than 10% polymorphic change for each polymorph after storage for 2 months at room temperature at 60% RH.

25. The stable mixture of claim 24, wherein the mixture undergoes less than 5% polymorphic change for each polymorph after storage for 2 months at room temperature at 60% RH.

26. The stable mixture of claim 25, wherein the mixture undergoes less than 1% polymorphic change for each polymorph after storage for 2 months at room temperature at 60% RH.

27. The stable mixture of any one of claims 17 to 26, wherein the dissolution rate in vitro of the stable mixture, when measured by the U.S.P. Paddle Method at 50-90 RPM in 900mL water is not less than 80% (by weight) of the mixture released after 30 minutes.

28. A pharmaceutical formulation comprising the stable mixture of any one of claims 17 to 27.

29. Use of a stable mixture according to any one of claims 17 to 27 in the preparation of a medicament for treating allergenic reactions in a mammal.

30. A process for preparing a stable mixture of crystalline form desloratadine I and II in a weight to weight ratio of 25% to 75% of either form, said process comprising:
a) combining desloratadine salt, toluene and a base to obtain a reaction mixture;
b) heating the mixture, whereby two phases are obtained;
c) separating the phases;
d) concentrating the separated organic phase;
e) dissolving the obtained concentrate in a toluene-2-propanol mixture containing less than 20% 2-propanol by volume;
f) cooling the solution to obtain a slurry;
g) combining the slurry with cold n-heptane; and
h) recovering mixture of desloratadine Forms I and II.

31. The process of claim 30, which further comprises washing the product of step c) with water.

32. The process of claim 30, which further comprises warming the product of step f) to 45°C.

33. Use of a stable mixture according to any one of claims 17 to 27 in the preparation of a pharmaceutical composition.

34. A process for preparing a pharmaceutical composition of desloratadine, said process comprising combining a stable mixture according to any one of claims 17 to 27 with a pharmaceutically acceptable excipient.

## Patentansprüche

1. Pharmazeutische Zusammensetzung von Desloratadin, umfassend ein Gemisch der kristallinen Formen I und II von Desloratadin in einem Gewichts-Gewichts-Verhältnis von 25% zu 75% einer der Formen zu der anderen und einen pharmazeutisch verträglichen Trägerstoff.

2. Pharmazeutische Zusammensetzung nach Anspruch 1, wobei das Verhältnis 50% beträgt.

3. Pharmazeutische Zusammensetzung nach Anspruch 1, wobei das Verhältnis 55 bis 65% von Form I zu 35 bis 45% von Form II beträgt.

4. Pharmazeutische Zusammensetzung von Desloratadin, umfassend die kristallinen Formen I und II von Desloratadin in einem Gewichts-Gewichts-Verhältnis von 20% bis 40% von Form II und 60% bis 80% von Form I und einen pharmazeutisch verträglichen Trägerstoff.

5. Pharmazeutische Zusammensetzung nach Anspruch 4, wobei der Gehalt an Form II in dem Gemisch 24% bis 38% beträgt.

6. Pharmazeutische Zusammensetzung nach einem der vorangegangenen Ansprüche, wobei das für die Zusammensetzung verwendete Gemisch eine Schmelztemperatur von 157°C bis 158°C, gemessen durch DSC, hat.

7. Pharmazeutische Zusammensetzung nach einem der vorangegangenen Ansprüche, wobei das für die Zusammensetzung verwendete Gemisch nach Mahlen für eine Minute zu weniger als 1 Gew.-% einer polymorphen Veränderung und chemischer Zersetzung unterliegt.

8. Pharmazeutische Zusammensetzung nach einem der vorangegangenen Ansprüche, wobei das für die Zusammensetzung verwendete Gemisch nach Lagerung bei 100% relativer Feuchte für eine Woche zu weniger als 1 Gew.-% einer chemischen Zersetzung unterliegt.

9. Pharmazeutische Zusammensetzung nach einem der vorangegangenen Ansprüche, wobei das für die Zusammensetzung verwendete Gemisch nach Lagerung für 2 Monate bei 40°C bei 75% RELATIVER FEUCHTE für jede polymorphe Form zu weniger als 10% einer polymorphen Veränderung unterliegt.

10. Pharmazeutische Zusammensetzung nach Anspruch 9, wobei das für die Zusammensetzung verwendete Gemisch nach Lagerung für 2 Monate bei 40°C bei 75% RELATIVER FEUCHTE für jede polymorphe Form zu weniger als 5% einer polymorphen Veränderung unterliegt.

11. Pharmazeutische Zusammensetzung nach Anspruch 10, wobei das für die Zusammensetzung verwendete Gemisch nach Lagerung für 2 Monate bei 40°C bei 75% RELATIVER FEUCHTE für jede polymorphe Form zu weniger als 3% einer polymorphen Veränderung unterliegt.

12. Pharmazeutische Zusammensetzung nach einem der vorangegangenen Ansprüche, wobei das für die Zusammensetzung verwendete Gemisch nach Lagerung für 2 Monate bei Raumtemperatur bei 60% RELATIVER FEUCHTE für jede polymorphe Form zu weniger als 10% einer polymorphen Veränderung unterliegt.

13. Pharmazeutische Zusammensetzung nach Anspruch 12, wobei das für die Zusammensetzung verwendete Gemisch nach Lagerung für 2 Monate bei Raumtemperatur bei 60% RELATIVER FEUCHTE für jede polymorphe Form zu weniger als 5% einer polymorphen Veränderung unterliegt.

14. Pharmazeutische Zusammensetzung nach Anspruch 13, wobei das für die Zusammensetzung verwendete Gemisch nach Lagerung für 2 Monate bei Raumtemperatur bei 60% RELATIVER FEUCHTE für jede polymorphe Form zu weniger als 1 % einer polymorphen Veränderung unterliegt.

15. Verwendung der pharmazeutischen Zusammensetzung nach einem der vorangegangenen Ansprüche bei der Herstellung eines Medikaments zur Verhinderung oder Behandlung allergischer Reaktionen in einem Säuger.

16. Verfahren zur Herstellung einer pharmazeutischen Zusammensetzung von Desloratadin, wobei das Verfahren die folgenden Stufen umfaßt:
a) Herstellen eines Gemischs der kristallinen Formen I und II von Desloratadin in einem Gewichts-Gewichts-Verhältnis von 20% bis 40% von Form II zu Form I und
b) Vereinigen des Gemischs mit einem pharmazeutisch verträglichen Trägerstoff unter Erhalt einer pharmazeutischen Zusammensetzung.

17. Stabiles Gemisch der kristallinen Formen I und II von Desloratadin in einem Gewichts-Gewichts-Verhältnis von 25% zu 75% jeweils einer der Formen zu der anderen.

18. Stabiles Gemisch der kristallinen Formen von Desloratadin in einem Gewichts-Gewichts-Verhältnis von 20-40% von Form II zu 60-80% von Form I.

19. Stabiles Gemisch nach Anspruch 18, wobei das Gewichts-Gewichts-Verhältnis von 24-38% von Form II zu 62-76% von Form I beträgt.

20. Stabiles Gemisch nach einem der Ansprüche 17 bis 19, wobei der Schmelzpunkt im Bereich von 157°C bis 158°C liegt.

21. Stabiles Gemisch nach einem der Ansprüche 17 bis 20, wobei das Gemisch dahingehend stabil ist, daß es nach Lagerung für 2 Monate ei 40°C bei 75% RELATIVER FEUCHTE für jede polymorphe Form zu weniger als 10% einer polymorphen Veränderung unterliegt.

22. Stabiles Gemisch nach Anspruch 21, wobei das Gemisch nach Lagerung für 2 Monate bei 40°C bei 75% RELATIVER FEUCHTE für jede polymorphe Form zu weniger als 5% einer polymorphen Veränderung unterliegt.

23. Stabiles Gemisch nach Anspruch 22, wobei das Gemisch nach Lagerung für 2 Monate bei 40°C bei 75% RELATIVER FEUCHTE für jede polymorphe Form zu weniger als 3% einer polymorphen Veränderung unterliegt.

24. Stabiles Gemisch nach einem der Ansprüche 17 bis 23, wobei das Gemisch nach Lagerung für 2 Monate bei Raumtemperatur bei 60% RELATIVER FEUCHTE für jede polymorphe Form zu weniger als 10% einer polymorphen Veränderung unterliegt.

25. Stabiles Gemisch nach Anspruch 24, wobei das Gemisch nach Lagerung für 2 Monate bei Raumtemperatur bei 60% RELATIVER FEUCHTE für jede polymorphe Form zu weniger als 5% einer polymorphen Veränderung unterliegt.

26. Stabiles Gemisch nach Anspruch 25, wobei das Gemisch nach Lagerung für 2 Monate bei Raumtemperatur bei 60% RELATIVER FEUCHTE für jede polymorphe Form zu weniger als 1% einer polymorphen Veränderung unterliegt.

27. Stabiles Gemisch nach einem der Ansprüche 17 bis 26, wobei die Auflösungsgeschwindigkeit des stabilen Gemischs in vitro, wenn sie durch das USP-Paddle-Verfahren bei 50-90 U.p.M. in 900 ml Wasser gemessen wird, derart ist, daß nach 30 Minuten nicht weniger als 80 (Gewichts-)% des Gemischs freigesetzt werden.

28. Pharmazeutische Formulierung, welche das stabile Gemisch nach einem der Ansprüche 17 bis 27 umfaßt.

29. Verwendung eines stabilen Gemischs nach einem der Ansprüche 17 bis 27 bei der Herstellung eines Medikaments zur Behandlung allergischer Reaktionen in einem Säuger.

30. Verfahren zur Herstellung eines stabilen Gemischs der kristallinen Formen I und II von Desloratadin in einem Gewichts-Gewichts-Verhältnis von 25% zu 75% jeweils einer der Formen zu der anderen, wobei das Verfahren folgendes umfaßt:
a) Vereinigen von Desloratadinsalz, Toluol und einer Base unter Erhalt eines Reaktionsgemischs,
b) Erhitzen des Gemischs, wodurch zwei Phasen erhalten werden,
c) Trennen der Phasen,
d) Konzentrieren der abgetrennten organischen Phase,
e) Lösen des erhaltenen Konzentrats in einem Toluol-2-Propanol-Gemisch, welches weniger als 20 Volumen-% 2-Propanol enthält,
f) Kühlen der Lösung unter Erhalt einer Aufschlämmung,
g) Vereinigen der Aufschlämmung mit kaltem n-Heptan und
h) Gewinnen des Gemischs der Formen I und II von Desloratadin.

31. Verfahren nach Anspruch 30, welches weiterhin das Waschen des Produkts aus Stufe c) mit Wasser umfaßt.

32. Verfahren nach Anspruch 30, welches weiterhin das Erwärmen des Produkts aus Stufe f) auf 45°C umfaßt.

33. Verwendung eines stabilen Gemischs nach einem der Ansprüche 17 bis 27 bei der Herstellung einer pharmazeutischen Zusammensetzung.

34. Verfahren zur Herstellung einer pharmazeutischen Zusammensetzung von Desloratadin, wobei das Verfahren das Vereinigen eines stabilen Gemischs nach einem der Ansprüche 17 bis 27 mit einem pharmazeutisch verträglichen Trägerstoff umfaßt.

## Revendications

1. Composition pharmaceutique de desloratadine comprenant un mélange de desloratadine de formes cristallines I et II selon un rapport en poids pour poids de 25% à 75% de l'une ou l'autre des formes à l'autre et un excipient acceptable du point de vue pharmaceutique.

2. Composition pharmaceutique selon la revendication 1, dans laquelle le rapport est de 50%.

3. Composition pharmaceutique selon la revendication 1, dans laquelle le rapport est de 55% à 65% de Forme I à 35% à 45% de Forme II.

4. Composition pharmaceutique de desloratadine comprenant les formes cristallines I et II de desloratadine selon un rapport en poids pour poids de 20% à 40% de Forme II et de 60% à 80% de Forme I, et un excipient acceptable du point de vue pharmaceutique.

5. Composition pharmaceutique selon la revendication 4, dans laquelle la teneur en Forme II du mélange est de 24% à 38%.

6. Composition pharmaceutique selon l'une quelconque des revendications précédentes, dans laquelle le mélange utilisé pour la composition a une température de fusion de 157°C à 158°C telle qu'elle est mesurée par DSC.

7. Composition pharmaceutique selon l'une quelconque des revendications précédentes, dans laquelle le mélange utilisé pour la composition subit un changement polymorphe et une dégradation chimique inférieurs à 1% en poids après un broyage d'une minute.

8. Composition pharmaceutique selon l'une quelconque des revendications précédentes, dans laquelle le mélange utilisé pour la composition subit une décomposition chimique inférieure à 1 % en poids après un stockage à une humidité relative de 100% pendant une semaine.

9. Composition pharmaceutique selon l'une quelconque des revendications précédentes, dans laquelle le mélange utilisé pour la composition subit un changement polymorphe inférieur à 10% pour chaque polymorphe après un stockage pendant 2 mois à 40°C et 75% d'humidité relative.

10. Composition pharmaceutique selon la revendication 9, dans laquelle le mélange utilisé pour la composition subit un changement polymorphe inférieur à 5% pour chaque polymorphe après un stockage pendant 2 mois à 40°C et 75% d'humidité relative.

11. Composition pharmaceutique selon la revendication 10, dans laquelle le mélange utilisé pour la composition subit un changement polymorphe inférieur à 3% pour chaque polymorphe après un stockage pendant 2 mois à 40°C et 75% d'humidité relative.

12. Composition pharmaceutique selon l'une quelconque des revendications précédentes, dans laquelle le mélange utilisé pour la composition subit un changement polymorphe inférieur à 10% pour chaque polymorphe après un stockage pendant 2 mois à la température ambiante et 60% d'humidité relative.

13. Composition pharmaceutique selon la revendication 12, dans laquelle le mélange utilisé pour la composition subit un changement polymorphe inférieur à 5% pour chaque polymorphe après un stockage pendant 2 mois à la température ambiante et 60% d'humidité relative.

14. Composition pharmaceutique selon la revendication 13, dans laquelle le mélange utilisé pour la composition subit un changement polymorphe inférieur à 1% pour chaque polymorphe après un stockage pendant 2 mois à la température ambiante et 60% d'humidité relative.

15. Utilisation de la composition pharmaceutique selon l'une quelconque des revendications précédentes, dans la préparation d'un médicament destiné à prévenir ou à traiter des réactions allergéniques chez un mammifère.

16. Procédé de préparation d'une composition pharmaceutique de desloratadine, ledit procédé comprenant les étapes de :
a) préparation d'un mélange de formes cristallines I et II de desloratadine selon un rapport en poids pour poids de 20% à 40% de la Forme II à la Forme I ; et
b) combinaison du mélange avec un excipient acceptable du point de vue pharmaceutique pour obtenir une composition pharmaceutique.

17. Mélange stable de formes cristallines I et II de desloratadine selon un rapport en poids pour poids de 25% à 75% de l'une ou l'autre des formes à l'autre.

18. Mélange stable de desloratadine de formes cristallines selon un rapport en poids pour poids de 20% à 40% de la Forme II à 60% à 80% de la Forme I.

19. Mélange stable selon la revendication 18, dans lequel le rapport en poids pour poids est de 24% à 38% de la Forme II à 62% à 76% de la Forme I.

20. Mélange stable selon l'une quelconque des revendications 17 à 19, dans lequel le point de fusion est compris dans la gamme de 157°C à 158°C.

21. Mélange stable selon l'une quelconque des revendications 17 à 20, dans lequel le mélange est stable en ce qu'il subit un changement polymorphe inférieur à 10% pour chaque polymorphe après un stockage pendant 2 mois à 40°C et 75% d'humidité relative.

22. Mélange stable selon la revendication 21, dans lequel le mélange subit un changement polymorphe inférieur à 5% pour chaque polymorphe après un stockage pendant 2 mois à 40°C et 75% d'humidité relative.

23. Mélange stable selon la revendication 22, dans lequel le mélange subit un changement polymorphe inférieur à 3% pour chaque polymorphe après un stockage pendant 2 mois à 40°C et 75% d'humidité relative.

24. Mélange stable selon l'une quelconque des revendications 17 à 23, dans lequel le mélange subit un changement polymorphe inférieur à 10% pour chaque polymorphe après un stockage pendant 2 moins à la température ambiante et 60% d'humidité relative.

25. Mélange stable selon la revendication 24, dans lequel le mélange subit un changement polymorphe inférieur à 5% pour chaque polymorphe après un stockage pendant 2 mois à la température ambiante et 60% d'humidité relative.

26. Mélange stable selon la revendication 25, dans lequel le mélange subit un changement polymorphe inférieur à 1% pour chaque polymorphe après un stockage pendant 2 mois à la température ambiante et 60% d'humidité relative.

27. Mélange stable selon l'une quelconque des revendications 17 à 26, dans lequel la vitesse de dissolution *in vitro* du mélange stable, lorsqu'elle est mesurée par la USP Paddle Method à 50-90 TPM dans 900 ml d'eau n'est pas inférieure à 80% en poids du mélange libéré après 30 minutes.

28. Formulation pharmaceutique comprenant le mélange stable selon l'une quelconque des revendications 17 à 27.

29. Utilisation d'un mélange stable selon l'une quelconque des revendications 17 à 27, dans la préparation d'un médicament destiné à traiter des réactions allergéniques chez un mammifère.

30. Procédé de préparation d'un mélange stable de formes cristallines I et II de desloratadine selon un rapport en poids pour poids de 25% à 75% de l'une ou l'autre forme, ledit procédé comprenant :
a) la combinaison d'un sel de desloratadine, de toluène et d'une base pour obtenir un mélange réactionnel ;
b) le chauffage du mélange de façon à obtenir deux phases ;
c) la séparation des phases ;
d) la concentration de la phase organique séparée ;
e) la dissolution du concentré obtenu dans un mélange de toluène et de 2-propanol contenant moins de 20% de 2-propanol en volume ;
f) le refroidissement de la solution pour obtenir une suspension ;
g) la combinaison de la suspension avec du n-heptane froid ; et
h) la récupération du mélange de Formes I et II de desloratadine.

31. Procédé selon la revendication 30, qui comprend de plus le lavage du produit de l'étape c) avec de l'eau.

32. Procédé selon la revendication 30, qui comprend de plus le chauffage du produit de l'étape f) à 45°C.

33. Utilisation d'un mélange stable selon l'une quelconque des revendications 17 à 27 dans la préparation d'une composition pharmaceutique.

34. Procédé de préparation d'une composition pharmaceutique de desloratadine, ledit procédé comprenant la combinaison d'un mélange stable selon l'une quelconque des revendications 17 à 27 avec un excipient acceptable du point de vue pharmaceutique.
